# EUROPEAN PATENT SPECIFICATION

(11) **EP 0 632 795 B1**
(45) Date of publication and mention of the grant of the patent: **07.01.1998**
(21) Application number: 93906203.0
(22) Date of filing: 23.02.1993
(51) Int. Cl.: C07C 2/62

(54) **AN ISOPARAFFIN-OLEFIN ALKYLATION CATALYST COMPRISING HF, SULFONE AND WATER**
EIN KATALYSATOR ZUR ALKYLIERUNG EINES ISOPARAFFINS MIT EINEM OLEFIN, WELCHER HF, SULFON UND WASSER ENTHÄLT
CATALYSEUR D'ALKYLATION D'UNE ISOPARAFFINE AVEC UNE OLEFINE COMPRENANT HF, SULFONE ET EAU

(30) Priority: 23.03.1992 US 856270
(43) Date of publication of application: 11.01.1995
(73) Proprietor: PHILLIPS PETROLEUM COMPANY, Bartlesville Oklahoma (US)
(72) Inventor: CHILD, Jonathan, Edward, Sewell, NJ 08080 (US); DEL ROSSI, Kenneth, Joseph, Woodbury, NJ 08096 (US)
(74) Representative: Dost, Wolfgang, Dr.rer.nat., Dipl.-Chem.
(86) International application number: US9301649
(87) International publication number: WO9319025

(56) References cited:
- EP-A- 0 568 090
- US-A- 2 267 730
- US-A- 2 448 400
- US-A- 3 766 293
- US-A- 3 778 489
- US-A- 3 795 712

## Description

The present invention relates to an isoparaffin: olefin alkylation process.

Alkylation is a reaction in which an alkyl group is added to an organic molecule. Thus an isoparaffin can be reacted with an olefin to provide an isoparaffin of higher molecular weight. Industrially, the concept depends on the reaction of a C₂ to C₅ olefin with isobutane in the presence of an acidic catalyst producing a so-called alkylate. This alkylate is a valuable blending component in the manufacture of gasolines due not only to its high octane rating but also to its sensitivity to octane-enhancing additives.

Industrial alkylation processes have historically used concentrated hydrofluoric or sulfuric acid catalysts under relatively low temperature conditions. Acid strength is preferably maintained at 88 to 94 weight percent by the continuous addition of fresh acid and the continuous withdrawal of spent acid. As used herein, the term "concentrated hydrofluoric acid" refers to an essentially anhydrous liquid containing at least about 85 weight percent HF.

Hydrofluoric and sulfuric acid alkylation processes share inherent drawbacks including environmental and safety concerns, acid consumption, and sludge disposal. For a general discussion of sulfuric acid alkylation, see the series of three articles by L. F. Albright et al., "Alkylation of Isobutane with C₄ Olefins", 27 Ind. Eng. Chem. Res., 381-397, (1988). For a survey of hydrofluoric acid catalyzed alkylation, see 1 Handbook of Petroleum Refining Processes 23-28 (R. A. Meyers, ed., 1986).

Hydrogen fluoride, or hydrofluoric acid (HF) is highly toxic and corrosive. Years of experience in its manufacture and use have shown that HF can be handled safely, provided the hazards are recognized and precautions taken. Though many safety precautions are taken to prevent leaks, massive or catastrophic leaks are feared primarily because the anhydrous acid will fume on escape creating a vapor cloud that can spread for a considerable distance. Previous workers in this field approached this problem from the stand- point of containing or neutralizing the HF cloud in the event of an accidental release.

U.S. Patents 4,938,935 and 4,985,220 to Audeh and Greco, as well as U.S. Patent 4,938,936 to Yan teach various methods for containing and/or neutralizing HF acid clouds following accidental release.

However, it would be particularly desirable to provide an additive which decreases the cloud forming tendency of HF without compromising its activity as an isoparaffin:olefin alkylation catalyst. Thus, in our International Publication No. WO 93/00314 we have described that the addition of sulfolane to HF can reduce its cloud forming tendency without significantly reducing its activity as an alkylation catalyst. However, it has been found that a mixture of HF and sulfolane is highly corrosive toward carbon steel which is a commonly used material of construction in HF alkylation process units. The problem of catalyst corrosivity is critical because the cost of retrofitting an existing HF alkylation process unit with corrosion-resistant alloy equipment could preclude replacing the concentrated HF catalyst with the safer HF/additive mixture. An object of the present invention is therefore to overcome or alleviate this problem.

In accordance with the present invention, it has been found that a mixture of hydrofluoric acid, sulfolane, and water is an effective isoparaffin:olefin alkylation catalyst, exhibits markedly reduced tendency to form vapor clouds upon release, and is surprisingly less corrosive than an anhydrous mixture of HF and sulfolane.

Thus the invention resides in a process for alkylation of an isoparaffin with an olefin in the presence of a catalyst composition comprising a mixture of hydrofluoric acid and sulfolane, wherein water is added to the mixture such that the mixture contains 0.5 to 10 weight percent water.

In a preferred embodiment, the catalyst composition comprises from 20 to 40 weight percent sulfolane and from 60 to 80 weight percent HF. The most preferred catalyst composition comprises from 25 to 35 weight percent sulfolane together with from 65 to 75 weight percent HF. It has surprisingly been discovered that the catalyst composition of the invention can be substituted for concentrated HF in a commercial riser-type alkylation process unit with only minimal equipment modifications. Controlling the water content within the range of from 0.5 to 10 weight percent, preferably from 1 to 5 weight percent, of the catalyst composition provides the additional unexpected benefit of decreasing the corrosivity of the catalyst toward carbon steel.

### Feedstocks

Feedstocks useful in the present alkylation process include at least one isoparaffin and at least one olefin. The isoparaffin reactant used in the present alkylation process has from 4 to 8 carbon atoms. Representative examples of such isoparaffins include isobutane, isopentane, 3-methylhexane, 2-methylhexane, 2,3-dimethylbutane and 2,4-dimethylhexane.

The olefin component of the feedstock includes at least one olefin having from 2 to 12 carbon atoms. Representative examples of such olefins include butene-2, isobutylene, butene-1, propylene, ethylene, hexene, octene, pentene, and heptene. The preferred olefins include the C₄ olefins, for example, butene-1, butene-2, isobutylene, or a mixture of two or more of these C₄ olefins, with butene-2 being the most preferred. Suitable feedstocks, for the process of the present invention are described in U.S. Patent 3,862,258 to Huang et al. at column 3, lines 44-56.

The molar ratio of isoparaffin to olefin is generally from 1:1 to 100:1, preferably from 1:1 to 50:1, and more preferably from 5:1 to 20:1.

### Catalyst Composition

The catalyst complex of the present invention comprises from 50 to 80 weight percent hydrofluoric acid together with from 20 to 50 weight percent sulfolane. Sulfolane (tetramethylene sulfone) has the formula:

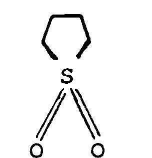

In a preferred embodiment, the catalyst composition comprises from 20 to 40 weight percent sulfolane and from 60 to 80 weight percent HF. The most preferred catalyst composition comprises from 25 to 35 weight percent sulfolane together with from 65 to 75 weight percent HF.

In accordance with the present invention, it has been found that a controlled amount of water surprisingly effects a dramatic reduction in the corrosivity of the catalyst composition. The catalyst composition suitably contains from 1 to 10 weight percent water, preferably from 1 to 5 weight percent water. While the most preferred water content varies slightly with the relative amounts of HF and sulfolane, the most preferred catalyst composition typically contains from 2 to 3 weight percent water.

### Process Conditions

The catalyst composition of the present invention may be readily substituted for the concentrated hydrofluoric acid catalyst in an existing hydrofluoric acid alkylation process unit, for example, a riser reactor alkylation process unit, without substantial equipment modifications. Accordingly, the conversion conditions for the process of the present invention resemble those of typical commercial hydrofluoric acid alkylation processes.

The present alkylation process is suitably conducted at temperatures of -18 to 66°C (0 to 150°F), preferably from 10 to 66°C (50 to 150°F), and more preferably from 21 to 43°C (70 to 110°F). Pressure is maintained to ensure a liquid phase in the alkylation reaction zone. Pressures typically range from 240 to 8400 kPa (20 to 1200 psig), preferably from 445 to 3550 kPa (50 to 500 psig). The reaction zone is preferably free from added hydrogen. Olefin feed rates generally range from 0.01 to 50 WHSV and more preferably from 0.5 to 20 hr⁻¹ WHSV. The mixed isoparaffin:olefin reactants may be contacted with the catalyst composition of the invention in any suitable reaction vessel, examples of which include stirred-tank reactors as well as riser-type reactors. Contact time for the mixed isoparaffin: olefin feed and the catalyst composition of the invention typically are within the range of from 0.1 second to 50 seconds, and are preferably from 8 seconds to 25 seconds.

The relative amounts of catalyst and reactants are defined herein by the acid-to-oil ratio. The volumetric acid-to-oil ratio (as used herein) is the ratio of the sum of the volumes of ASO (acid soluble oil), acid, and sulfolane to the total isoparaffin and olefin reactor feed. The volumetric acid-to-oil ratio typically falls within the range 0.1:1 to 10:1, preferably from 0.1:1 to 5:1. contact time between the catalyst and the isoparaffin:olefin feedstock typically ranges from 1 to 50 seconds, preferably from 8 to 25 seconds.

The sulfolane component of the alkylation catalyst composition may be added by injection directly into the alkylation process unit, or may be mixed with the hydrocarbon charge, or may be mixed with the fresh and/or the circulating acid catalyst component, or with a stream of mixed acid/additive catalyst. Downstream from the alkylation reaction zone, the sulfolane is preferably separated from the alkylate product stream, mixed with fresh and/or circulating acid and/or circulating acid/additive catalyst mixture, and recycled to the alkylation reaction zone.

The invention will now be more particularly described with reference to the Examples and the accompanying drawings, in which:
Figure 1a is a plot of TMP/DMH ratio (trimethylpentane/dimethylhexane) as a function of weight percent sulfolane in HF for isoparaffin:olefin alkylation at 27°C (80°F), 1310 kPa (175 psig), 10/1 isobutane/butene ratio and olefin WHSV of 0.3 hr⁻¹;
Figure 1b is a plot of C₉+ byproduct in the alkylate product as a function of weight percent sulfolane in HF under the conditions set forth with reference to Figure 1a;
Figure 1c is a plot of the MON (Motor Octane Number) of the C₅+ portion of the alkylate product produced by the isoparaffin: olefin reaction (described above with reference to Figure 1a) as a function of weight percent sulfolane in HF;
Figure 2 is a plot of vapor pressure at 24°C (76°F) as a function of weight percent sulfolane in HF;
Figure 3 is a plot of percent rainout (weight) as a function of vapor pressure for neat sulfolane, neat HF, and mixtures of HF and sulfolane containing from 20 to 80 percent sulfolane (weight); and
Figure 4 shows the effect of added water on the corrosion rate (plotted on the y-axis) of carbon steel exposed to a solution of sulfolane in hydrofluoric acid as a function of the water content (plotted on the x-axis) of the HF/sulfolane solution.

### EXAMPLE 1

Anhydrous HF (40 grams, obtained from Matheson Chemical Company of Bridgeport, New Jersey) was condensed into a clean, dry autoclave (1000 cc). Isobutane (100 grams) was added, and the autoclave was stirred at 1500 rpm. The autoclave was brought to room temperature (22°C, 71°F) and pressurized to 790 kPa (100 psig). A pre-mixed 10:1 weight:weight mixture of isobutane: 2-butene feed (obtained from Matheson Chemical Company) was added at a rate of 250 cc/hour for 2 hours under autogeneous pressure for a total isobutane:2-butene charge of 500 cc. A 10-15°F (6-8°C) temperature rise was observed during feed addition resulting in an average reaction temperature of 27-30°C (80-85°F). The autoclave was sampled (300 cc) immediately after feed addition was complete. The sample was flashed at room temperature and quenched with a chilled water trap. Samples of the liquid and gas products were analyzed by capillary GC (60m DB-1 column). The results of Example 1 are shown in the Table A below.

### EXAMPLES 2-4

The following procedure was followed for Examples 2-4. In a typical experiment, 10 grams of sulfolane (tetramethylene sulfone, Phillips Petroleum Co.) was loaded into a clean, dry 1000 cc autoclave. Sulfolane was stored in a vacuum desiccator over P₂O₅ prior to use. The autoclave was sealed and cooled with liquid nitrogen. The autoclave was evacuated and 40 grams of anhydrous HF (Matheson) were condensed into the autoclave. The HF/sulfolane mixture was warmed to room temperature (22°C). Isobutane (100 grams) was added to the mixture, the autoclave was pressurized to 790 kPa (100 psig) and stirred at 1500 rpm. A pre-mixed 10/1 wt/wt isobutane/2-butene feed (Matheson) was then introduced at 250 cc/hr. A 3-6°C (5-10°F) temperature rise was typically observed during reaction. After two hours, feed addition was halted and a 300 cc liquid sample was obtained. The liquid sample was depressured through an ice cooled trap (filled with 50 cc of water) which was connected to a gas sampling bomb and wet test meter. The liquid alkylate product and gas sample were analyzed with a Varian 6000 gas chromatograph equipped with a 60 meter DB-1 capillary column.

Table A below lists the results with HF/sulfolane mixtures containing up to 50 wt% sulfolane in HF. With 20 wt% sulfolane in HF (125 moles HF per liter sulfolane), performance was comparable to pure HF. The ratio of high octane trimethylpentanes to lower octane dimethylhexanes (TMP/DMH) was 9.4 with 80/20 HF/sulfolane compared to 9.2 with pure HF. Performance diminished slightly upon adding 50 wt% sulfolane to HF (63 moles HF per liter of sulfolane). Alkylate with 50/50 HF/sulfolane catalyst had a TMP/DMH ratio of 6.5 and contained 11.8 wt% C₉+. A 40/60 HF/sulfolane catalyst (42 moles of HF per liter of sulfolane) showed no activity for alkylation. The only observed product was butyl fluoride formed by hydrofluorination of 2-butene feed. Thus, the useful concentration range for sulfolane in HF was shown to be below about 60 wt% in HF (greater than about 40 moles of HF per liter of sulfolane).

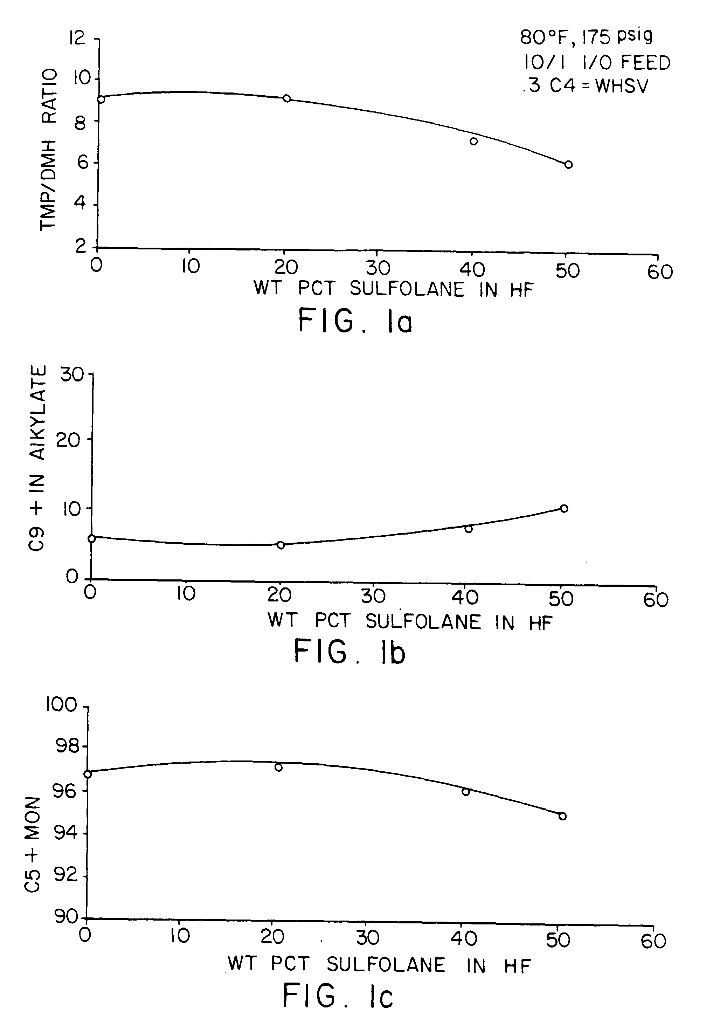

HF/sulfolane catalyst performance is plotted as a function of sulfolane loading in Figures 1a, 1b, and 1c. Alkylate quality increased slightly upon adding 20 wt% sulfolane to HF (C₅+ MON 97.5 vs 97 for pure HF), then decreased with further sulfolane dilution. Activity for isoparaffin/olefin alkylation was not observed above about 50 wt% sulfolane in HF.

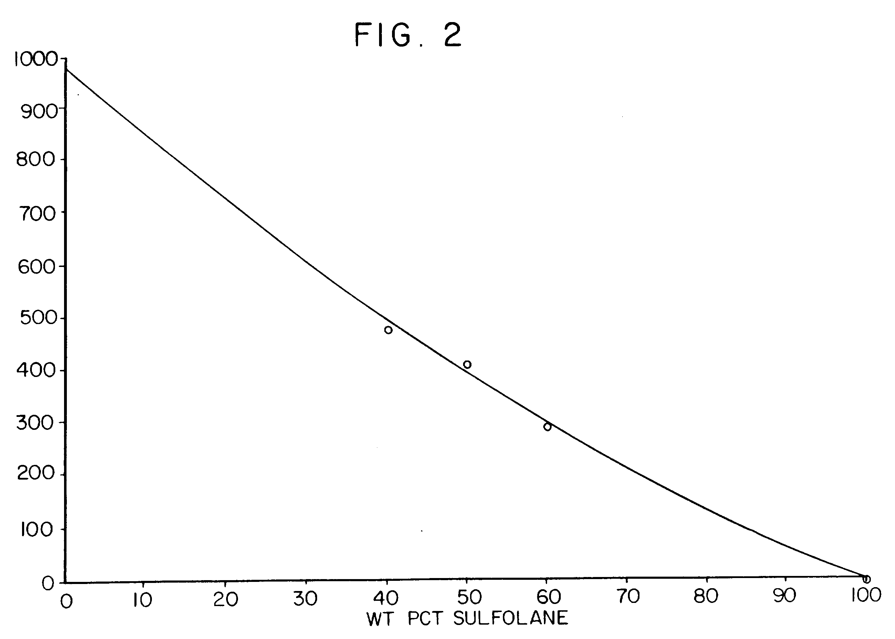

Vapor pressures (Torr) for HF/sulfolane mixtures are plotted as a function of sulfolane loading in Figure 2, showing that diluting HF with sulfolane dramatically reduces vapor pressure.

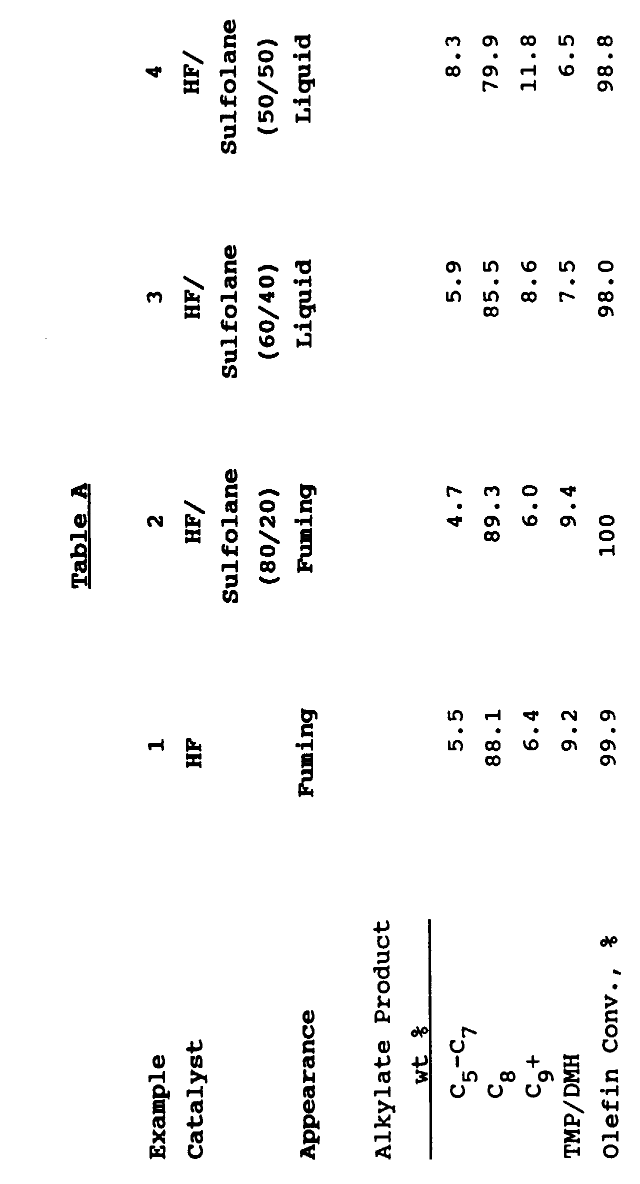

### Examples 5-7

HF/sulfolane mixtures were evaluated in a 46 cm (1.5 foot) long riser reactor having total volume of about 120 cc. The reactor was first filled with liquid isobutane at approximately 1000 kPa (130 psig). Approximately 100 cc of premixed HF/sulfolane catalyst (70/30 wt/wt for Example 5, 60/40 wt/wt for Examples 6 and 7) was pressured into the reactor from the bottom, displacing a portion of the isobutane. The isobutane flow was maintained to keep the injection nozzle clear. Hydrocarbon residence time in the riser was approximately 6 seconds with a hydrocarbon drop diameter of about 300 microns. The olefin feed was obtained from a commercial HF alkylation process unit and was characterized by the following average composition:

| Component | Weight Percent |
|---|---|
| C₃= | 12.4 |
| C₄= | 81.8 |
| C₅= | 5.8 |
| Total: | 100.0 |

Conversion conditions and results for Example 5 are shown below in Table B.

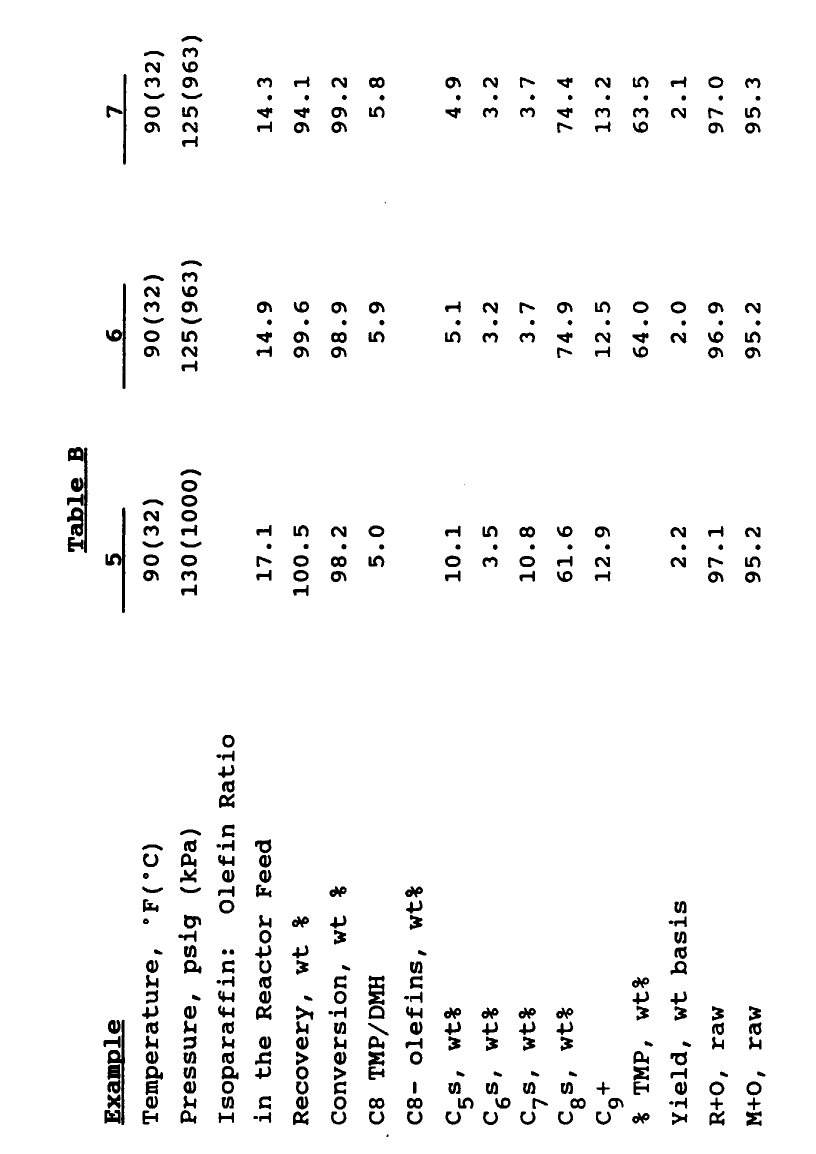

### Examples 8-15

Examples 8-15 examined the cloud-forming tendency of various mixtures of HF and sulfolane. The cloud-forming tendency was measured in terms of rainout, which is defined as the fraction of the released substance which falls to the ground within a defined area downwind from the point of release. For example, if 50% (weight) of a released substance falls to the ground within the defined area downwind from the point of release, the rainout is 50% (weight). Rainout is inversely related to cloud-forming tendency. In the event of an accidental release, material which precipitates or rains out cannot pose a toxic hazard downwind from the site of the accidental release.

The HF and sulfolane were mixed in a 2-liter stirred autoclave which was fabricated from 316 stainless steel and containing a cooling/heating coil. The autoclave was pressured with nitrogen, and the contents of the autoclave were then released through a nozzle into a flow chamber. The liquid rainout was collected in trays and the material which carried over was scrubbed with water in accordance with the procedure set forth below.

The sulfolane was weighed and placed into the 2-liter autoclave. Anhydrous HF from a cylinder was measured using a calibrated Kel-F lined sight glass and pressured into the autoclave. The amount of HF used per run varied from 109 to 434 grams. Once the material was loaded into the autoclave, the wind velocity in the flow chamber was adjusted to the desired setting, usually about 0.9 m/s to simulate a typical release wind speed. Ambient humidity was used. The autoclave mixer was also turned on for several minutes to mix the contents. Water was placed into each of three dropout trays to catch the precipitated HF and sulfolane as well as to minimize evaporative losses. The water-filled trays were weighed to determine the pre-release base line weights.

The autoclave pressure was then set with nitrogen and the autoclave temperature was set by warm water or a Neslab cooler. The valve to the release orifice was then opened, and the material flowed into the chamber. The orifice diameter was 0.635 mm. The dimensions of the flow chamber were approximately 100 cm (40 in) length by 30 cm (12 in) height by 15 cm (6 in) depth. Because the release material has sufficient inertia to travel a greater length than the flow chamber, an impingement plate was placed at the end of the third tray. This plate was covered with steel wool to minimize splashing and to produce a good rainout of 98 percent with the base case water runs.

The liquid rainout was collected in the trays, with most of the material going into the tray closest to the impingement plate. The trays were then weighed to determine how much material rained-out. The data were then corrected for evaporation of water from the trays, liquid hold-up in the impingement plate pad, residual material in the autoclave, and material that adhered to the walls of the chamber.

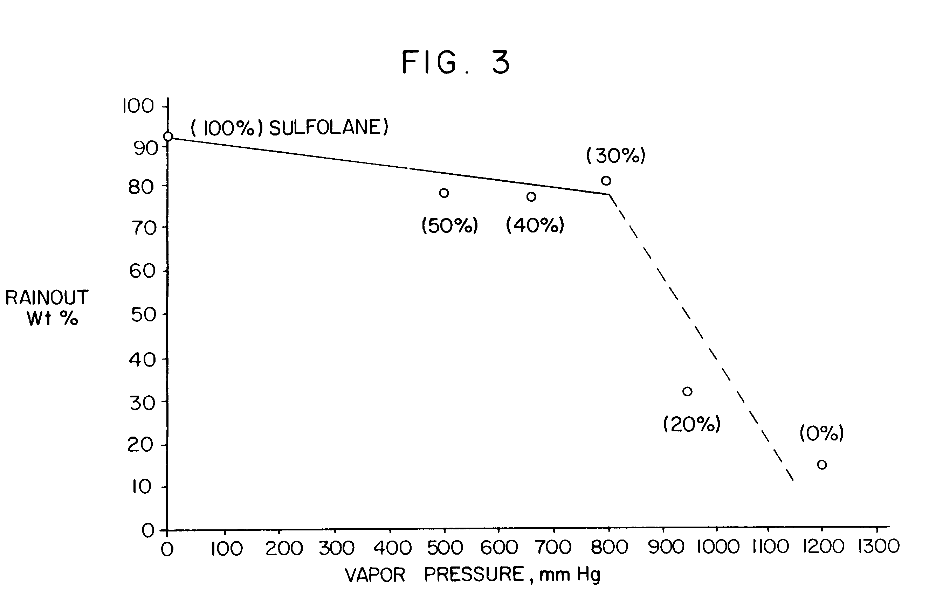

The rainout data are shown below in Table C, and plotted in Figure 3. Table C also list the corresponding C₅+ alkylate TMP (trimethylpentane) content produced with each of the HF/sulfolane mixtures as isobutane: butene alkylation catalyst. These results show that the preferred catalyst composition provides excellent rainout (>70 wt.%) while also producing a high quality alkylate product.

**Table C**

| Conditions: 450 kPa (50 psig), 32°C (90°F), 0.635 mm orifice diameter. | | | |
|---|---|---|---|
| Example No. | Sulfolane Wt % | TMP Wt % | Rainout, wt % by Material Balance |
| 8 | 60 | * | |
| 9 | 50 | | 76 |
| 10 | 50 | 69 | 76 |
| 11 | 50 | | 82 |
| 12 | 40 | 73 | 76 |
| 13 | 40 | | 78 |
| 14 | 30 | 78 | 81 |
| 15 | 20 | 80 | 32 |

| | | | |
|---|---|---|---|
| * Less than 5 wt %. | | | |

### EXAMPLES 16-19

Mixtures of HF and sulfolane were evaluated for their corrosivity toward carbon steel in accordance with the following procedure.

Sulfolane (tetramethylene sulfone as received from Phillips Petroleum Company) was vacuum distilled twice from KOH and once from CaH₂. The dried sulfolane was stored in a vacuum dessicator over P₂O₅.

In a typical experiment, a carbon steel rod, 13 cm (5.25") long and 0.6 cm (0.25") diameter, was attached to a teflon connector to the top of a 300 cc stainless steel autoclave (fabricated by Autoclave Engineers, Inc.). Purified sulfolane (49.5 grams) and deionized water (1 gram) were added to the autoclave under a nitrogen atmosphere. The autoclave was sealed, cooled with liquid nitrogen, and evacuated with a rough vacuum. HF (60 grams, Matheson) was condensed into the autoclave. The cold autoclave was connected to a pilot unit equipped with a LPG feed system and acid scrubber. The autoclave was warmed to 29°C (85°F), pressurized to 790 kPa (100 psig) with isobutane (Matheson), and stirred at 100 rpm. The contents were purged with 280 cc/hr of isobutane for 24 hours. The isobutane purge was removed and the autoclave was sealed for 150 hours at 29°C (85°F) and 1790 kPa (100 psig). HF loss during the isobutane purge was estimated at 10 grams, thus the purged mixture sealed for 150 hours was roughly 50/49/1 wt./wt./wt. HF/sulfolane/H₂O.

At the end of the corrosion experiment, the autoclave was vented through caustic scrubbers, and opened in a fume hood. The carbon steel rod was removed, rinsed with acetone and stored in a dessicator. The bottom portion (2.5 cm) of the carbon steel rod that was immersed in the HF/sulfolane catalyst developed a protective coating. The protective film was removed with a mechanical bead blaster to expose the non-corroded metal. The corrosion rate was determined from the measured decrease in rod thickness.

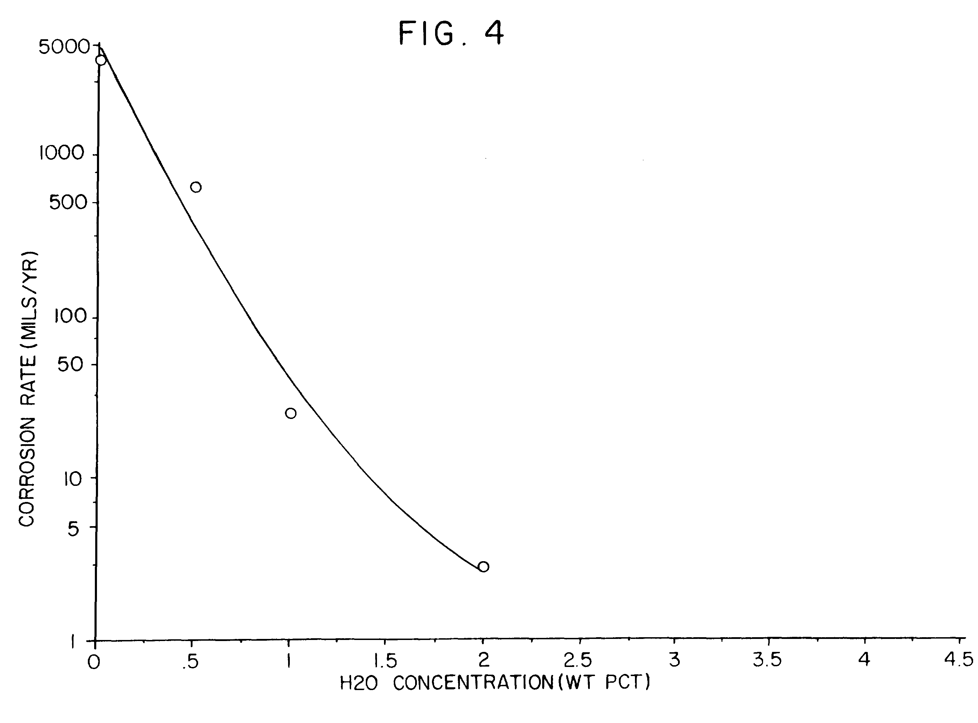

Results from Examples 16-19 are shown in Table D. A graph of corrosion rate as a function of water content is shown in Figure 4.

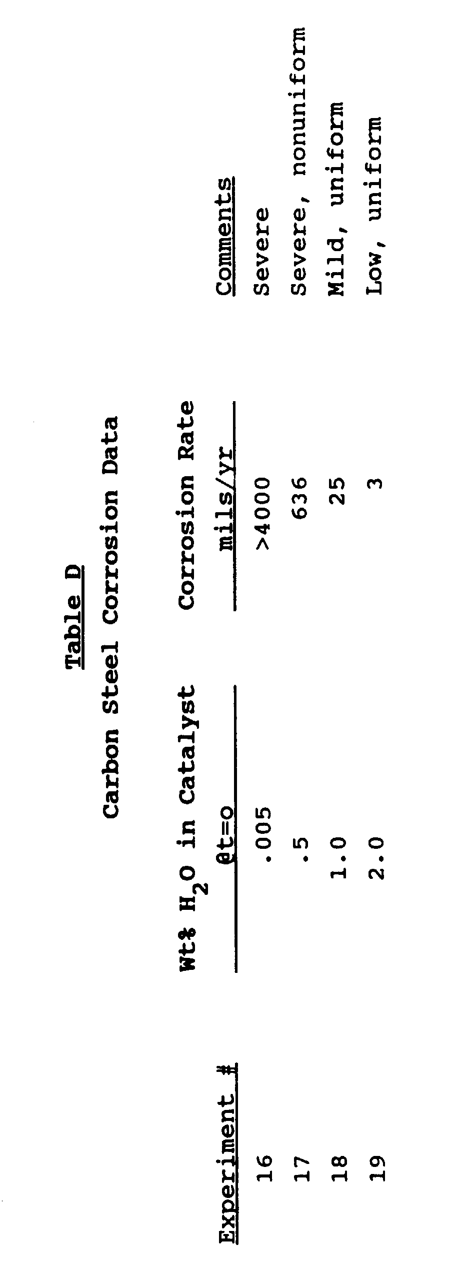

The decrease in corrosion rate with the addition of a controlled amount of water is surprising and unexpected because it is well known in the refining industry that anhydrous HF can be contained in carbon steel, but that wet HF aggressively corrodes carbon steel and must be contained in a nickel-enriched corrosion resistant alloy such as Monel 400 brand alloy.

## Claims

1. A process for alkylation of an isoparaffin with an olefin in the presence of a catalyst composition comprising a mixture of hydrofluoric acid and sulfolane, wherein water is added to the mixture such that the mixture contains 0.5 to 10 weight percent water.

2. The process of claim 1 wherein the catalyst composition comprises from 10 to 50 weight percent sulfolane.

3. The process of claim 1 wherein the catalyst composition comprises from 20 to 40 weight percent sulfolane.

4. The process of claim 1 wherein the catalyst composition comprises from 25 to 35 weight percent sulfolane.

5. The process of claim 1 comprising adding to the mixture from 1 to 5 weight percent water.

6. The process of claim 1 comprising adding to the mixture from 2 to 3 weight percent water.

7. The process of claim 1 conducted at a temperature of -18 to 66°C (0 to 150°F) and a pressure of 240 to 8400 kPa (20 to 1200 psig).

8. The process of claim 1 conducted at a temperature of 10 to 66°C (50 to 150°F) and a pressure 445 to 3550 kPa (50 to 500 psig).

## Patentansprüche

1. Verfahren zur Alkylierung eines Isoparaffins mit einem Olefin in Gegenwart einer Katalysatorzusammensetzung, die ein Gemisch aus Fluorwasserstoffsäure und Sulfolan enthält, wobei dem Gemisch Wasser in einer solchen Menge zugesetzt wird, daß das Gemisch 0,5 bis 10 Gew.-% Wasser enthält.

2. Verfahren nach Anspruch 1, wobei die Katalysatorzusammensetzung 10 bis 50 Gew.-% Sulfolan enthält.

3. Verfahren nach Anspruch 1, wobei die Katalysatorzusammensetzung 20 bis 40 Gew.-% Sulfolan enthält.

4. Verfahren nach Anspruch 1, wobei die Katalysatorzusammensetzung 25 bis 35 Gew.-% Sulfolan enthält.

5. Verfahren nach Anspruch 1, umfassend die Zugabe von 1 bis 5 Gew.-% Wasser zum Gemisch.

6. Verfahren nach Anspruch 1, umfassend die Zugabe von 2 bis 3 Gew.-% Wasser zum Gemisch.

7. Verfahren nach Anspruch 1, durchgeführt bei einer Temperatur von -18 bis 66°C (0 bis 150°F) und einem Druck von 240 bis 8400 kPa (20 bis 1200 psig).

8. Verfahren nach Anspruch 1, durchgeführt bei einer Temperatur von 10 bis 66°C (50 bis 150°F) und einem Druck von 445 bis 3550 kPa (50 bis 500 psig).

## Revendications

1. Un procédé d'alkylation d'une isoparaffine avec une oléfine en présence d'une composition de catalyseur comprenant un mélange d'acide fluorhydrique et de sulfolane, dans lequel de l'eau est ajoutée au mélange si bien que le mélange contient de 0,5 à 10 % en poids d'eau.

2. Le procédé selon la revendication 1, dans lequel la composition de catalyseur comprend de 10 à 50 % en poids de sulfolane.

3. Le procédé selon la revendication 1, dans lequel la composition de catalyseur comprend de 20 à 40 % en poids de sulfolane.

4. Le procédé selon la revendication 1, dans lequel la composition de catalyseur comprend de 25 à 35 % en poids de sulfolane.

5. Le procédé selon la revendication 1 comprenant l'addition au mélange de 1 à 5 % en poids d'eau.

6. Le procédé selon la revendication 1 comprenant l'addition au mélange de 2 à 3 % en poids d'eau.

7. Le procédé selon la revendication 1 réalisé à une température de -18 à 66°C (0 à 150°F) et sous une pression de 240 à 8400 kPa (20 à 1200 psig).

8. Le procédé selon la revendication 1 réalisé à une température de 10 à 66°C (50 à 150°F) et sous une pression de 445 à 3550 kPa (50 à 500 psig).
